# EUROPEAN PATENT APPLICATION

(11) **EP 0 676 642 A1**
(43) Date of publication of application: **11.10.1995**
(21) Application number: 95105024.4
(22) Date of filing: 04.04.1995
(51) Int. Cl.: G01N 33/92, C12Q 1/60

(54) **Method for quantitatively analyzing a component in a lipoprotein fraction**

(30) Priority: 05.04.1994 JP 66998/94
(71) Applicant: INTERNATIONAL REAGENTS CORPORATION, Kobe-shi Hyogo-ken (JP)
(72) Inventor: Hashiguchi, Yoichi, c/o International Reagents, Nishi-ku, Kobe-shi, Hyogo-ken (JP); Ikeda, Masafumi, c/o International Reagents, Nishi-ku, Kobe-shi, Hyogo-ken (JP); Kakuyama, Tsutomu, c/o International Reagents, Nishi-ku, Kobe-shi, Hyogo-ken (JP)
(74) Representative: VOSSIUS & PARTNER

(57) **Abstract**

A component such as cholesterol contained in a specific lipoprotein fraction in a biological sample is quantitatively analyzed by agglutinating the specific lipoprotein fraction, leading a component, which is contained in lipoprotein fractions other than the specific lipoprotein fraction and is the same as the component to be analyzed, to a different reaction system which does not take part in the quantitative analysis; dissolving the once agglutinated specific lipoprotein fraction, subjecting the specific lipoprotein fraction to a quantitative reaction, and measuring a degree of a change caused by the quantitative reaction to determine an amount of the component in the specific lipoprotein fraction.

## Description

The present invention relates to a method for quantitatively analyzing a component contained in a specific lipoprotein fraction which is present in a biological sample such as blood serum, blood plasma and the like, in particular, cholesterol contained in the LDL fraction for the purpose of the use in a clinical test.

In blood, a lipid is bound with an apoprotein to form a lipoprotein and metabolized. The lipoproteins are classified according to their densities into various fractions such as chylomicron (CM), very low density lipoprotein (VLDL), intermediate density lipoprotein (IDL), low density lipoprotein (LDL), and high density lipoprotein (HDL). Various diseases and factors have influences on the metabolism of these lipoproteins to cause the increase or decrease of the lipoprotein fractions in the blood.

It is known that, among others, LDL takes part in accumulation of cholesterol on inner walls of blood vessels and has an arteriosclerosis function. The cholesterol in the LDL fraction is in general called as "bad cholesterol" and its presence or concentration is measured for prevention or diagnosis of arteriosclerosis, ischemic heart diseases and the like.

Hitherto, for the measurement of the LDL fraction, there are known precipitation, ultracentrifuge, electrophoresis, etc. In the precipitation method, the LDL fraction is precipitated by a precipitant such as a polyanion to separate the LDL fraction, and the LDL fraction is assayed. But, the procedures of the sedimentation are very complicated as in the ultracentrifuge and electrophoresis methods, it cannot be employed in an automatic analyzer which is widely and daily used in the clinical test field. Japanese Patent KOKAI Publication No. 45562/1983 discloses a precipitation method using polyvinyl sulfate having a monovalent cation, but this method cannot be employed in the automatic analyzer.

As components contained in the specific fraction, various components are confirmed in the clinical test field. In particular, cholesterol, triglyceride and lipoprotein are well known. Among them, when cholesterol is assayed, so-called enzyme methods, which are performed under mild conditions, have a high specificity of the reaction and use an enzymatic reaction, are exclusively used.

The enzyme methods are roughly classified into a method for measuring an absorbance in a visible light range by combining cholesterol esterase (CE) and cholesterol oxidase (CO) with peroxidase (POD) and a color former, and a method for measuring an absorbance in a UV light range by combining CE and cholesterol dehydrogenase (CHD) with a co-enzyme.

As described above, the conventional methods for analyzing the LDL fraction cannot be employed in the automatic analyzer. That is, since the above precipitation method requires a collection step of a sample by centrifuge separation, it cannot be multichanneled by combining the cholesterol analysis with other test items in the automatic analyzer which uses the sample such as the blood serum as it is. In addition, when the supernatant is collected in the centrifuge separation, there is some danger that a lower layer may be contaminated.

Objects of the present invention are to quantitatively analyze a component contained in a specific lipoprotein fraction which is present in a biological sample with an automatic analyzer by solving the above problems and, in particular, to provide a useful quantitative analysis method of cholesterol in the LDL fraction in the clinical test.
Fig. 1 shows a typical analysis pattern in Example 1, and
Fig. 2 is a graph showing the results obtained in Example 2.

As a result of the extensive study, it has been found that, when the specific lipoprotein fraction such as the LDL fraction is agglutinated, and a component, which is contained in lipoprotein fractions other than the specific lipoprotein fraction and is the same as the component that is contained in the specific fraction and to be analyzed, is isolated in a different reaction system from a reaction system for the quantitative analysis, the specific fraction is not involved in the different reaction since it is agglutinated.

The further study revealed that, when the once agglutinated specific fraction is dissolved to an extent that it can participate in the reaction for the quantitative analysis, the reaction for the quantitative analysis can be carried out, and that, in some cases, the agglutinated specific fraction is optionally further dissolved to an extent that the reaction for the quantitative analysis can be carried out and the reaction can be terminated. Then, some change which is caused by the reaction, that is, a change of a concentration of the component or a reagent which reacts with the component or a concentration of a material formed by the reaction such as a light absorbing or emitting material is measured.

According to the present invention, there is provided a method for the direct quantitative analysis of a component contained in a specific lipoprotein fraction which is present in a biological sample, comprising steps of:
agglutinating said specific lipoprotein fraction,
leading a component, which is contained in lipoprotein fractions other than said specific lipoprotein fraction and is the same as said component that is contained in said specific lipoprotein fraction and to be analyzed, to a different reaction system which does not take part in said quantitative analysis;
dissolving said once agglutinated specific lipoprotein fraction,
subjecting said specific lipoprotein fraction to a quantitative reaction,
and
measuring a degree of a change caused by said quantitative reaction to determine an amount of said component in said specific lipoprotein fraction.

To agglutinate the specific fraction, a agglutinant or an antibody is preferably used.

Herein, the agglutinant intends to mean a material which causes agglutination through a chemical reaction, while the antibody is an antibody against the specific lipoprotein fraction and causes an immunoagglutination. The agglutinant or the antibody may be used independently or in combination according to the kind of fraction to be agglutinated.

When they are used in combination, for example, the agglutinant may be first added and then the antibody may be added, or they may be added at the same time.

As the agglutinant, any of agglutinants which can achieve the objects of the present invention may be used. For example, the agglutinants used in the above described precipitation methods or other agglutinants can be used.

For example, to precipitate the LDL fraction in the blood, polyethylene glycol (PEG), phosphotungstic acid, dextran sulfate, heparin and the like are used solely or in combination with a cation such as Mg⁺⁺, Mn⁺⁺, Ca⁺⁺, Li⁺⁺, Ni⁺⁺, etc.

As the antibody, any antibody that achieves the objects of the present invention may be used. For example, to immunoagglutinate the LDL fraction, one or more of polyclonal or monoclonal anti-bodies against LDL, apo-B or β-lipoprotein may be used.

As the reagent for detecting the component contained in the specific lipoprotein fraction, any of conventional reagents used in the clinical test field may be used.

For example, to quantitatively analyze the cholesterol, a reagent comprising CE, CO and POD, which is used in the above enzyme method, is preferably used.

A reaction time of the reaction between the sample and the reagent till the termination of the reaction varies with the reagent to be used. While, with some reagents, the quantitative result obtained when the reaction is terminated in the course of the reaction is the same as that obtained after the reaction is completed, the reaction time is usually from 1 to 60 minutes. When the combination of CE, CO and POD is used for the quantitative analysis of the cholesterol, the reaction time is usually from 2 to 30 minutes.

To terminate the reaction, any conventional method may be used. For example, in the enzyme method, an inhibitor which inhibits the enzyme is used.

The inhibitor is known and may be selected from the group of heavy metals, antiseptics, protein-denaturants, surfactants and the like. For the quantitative analysis of the cholesterol using CE, CO and POD, examples of the inhibitor are sodium azide, guanidine hydrochloride, and the like.

Instead of terminating the quantitative reaction, the amount of the component may be measured after the reaction saturates. Alternatively, the amount of the component is measured after a specific time period, and then the whole amount of the component is calculated from a beforehand prepared calibration curve.

To lead the lipoprotein fractions other than the specific lipoprotein fraction to the different reaction system, various reactions may be used.

For example, when the combination of CE, CO and POD is used to measure the amount of cholesterol in the LDL fraction, a reagent comprising the necessary enzyme but not containing a part of a color former is reacted with the sample to form a colorless complex is formed. Thereafter, when all of CE, CO, POD and the color former are reacted with the sample, the already formed colorless complex does not participate in this latter reaction.

As an example, when CE, CO, POD and HDAOS [N-(2-hydroxy-3-sulfopropyl)-3,5-dimethoxyaniline] as a color former are reacted with the sample after the agglutination of the specific lipoprotein fraction, the cholesterol ester and cholesterol in the lipoprotein fractions other than the specific lipoprotein fraction generate hydrogen peroxide by the action of CE and CO, and hydrogen peroxide reacts with POD and HDAOS to form a colorless complex. At this stage, since the specific lipoprotein fraction is agglutinated, it does not involved in this reaction. Thereafter, the specific lipoprotein is dissolved to an extent that CE and CO can react with cholesterol and its ester, and a reagent comprising CE, CO, POD, HDAOS and 4-aminoantipyrin is added. Then, the cholesterol ester and cholesterol in the specific lipoprotein fraction generate hydrogen peroxide by the action of CE and CO, and hydrogen peroxide reacts with POD, 4-aminoantipyrin and HDAOS to form a pigment having the maximum absorbance at a wavelength around 580 nm. At this stage, if necessary, the fraction is further dissolved to an extent that the absorbance can be measured, and the absorbance is measured to quantitatively measure the content of cholesterol in the specific lipoprotein fraction.

As other example, it is possible to use a reaction system which is different from the quantitative reaction and does not take part in the quantitative reaction.

For instance, when the enzymatic reaction comprising CE, CO and POD is used as the quantitative reaction, the reaction system of CE, CHD and NAD⁺ is reacted with the lipoprotein fractions other than the specific lipoprotein fraction to generate NADH. Thereafter, CHD is inhibited to stop this reaction, and the specific lipoprotein fraction is dissolved to the extent that CE and CO can function. Then a quantitative analysis reagent containing CE, CO, POD, HDAOS and 4-aminoantipyrin is reacted with the sample, the specific lipoprotein fraction is further dissolved, if necessary, to the extent that the absorbance caused by this reaction can be measured, and the absorbance is measured as described above to quantitatively measure the content of cholesterol.

To dissolve the once agglutinated specific lipoprotein fraction, a solubilizer is used. As the solubilizer, surfactants, inorganic salts and so on may be used independently or in combination thereof. Preferred examples of the solubilizer are TRITON X, ADECATOL, sodium chloride and potassium chloride.

For example, when the once agglutinated is dissolved to the extent that cholesterol is quantitatively measured, TRITON X-100 is preferably used.

To further dissolve the agglutinated specific lipoprotein fraction, protein-denaturants, surfactants, inorganic salts and the like are used independently or in combination of two or more of them. Preferred examples of the solubilizing agent used in this step are guanidine and its salts such as hydrochloride.

In the method of the present invention, since the specific lipoprotein fraction such as the LDL fraction is agglutinated and dissolved later, it is possible to measure the component by the multi-channel analysis using the automatic analyzer in the clinical test. Such multichannel analysis has been difficult by the conventional method.

Since the known enzyme can be used as the reagent for quantitative analysis, the reaction time can be shortened, and multi-sample processing is possible.

In addition, since the conventional precipitation method requires an excessive amount of the sample because the supernatant after centrifuge separation is used as the test sample, a large amount of the sample such as the blood serum is necessary. In the present invention, since the sample is directly used as the test sample, the amount of the sample can be decreased, and the method of the present invention is suitable for the simultaneous multi-item analysis.

Further, in the present invention, since the optical measurement can be used, the method of the present invention can be used in various analyzers which are widely used in the clinical test field. Therefore, the present invention is very useful in the clinical test.

The present invention will be explained further in detail by the following Examples, which do not limit the scope of the invention in any way.

### Example 1

This example shows an embodiment of the quantitative analysis of the cholesterol in the LDL fraction according to the present invention.

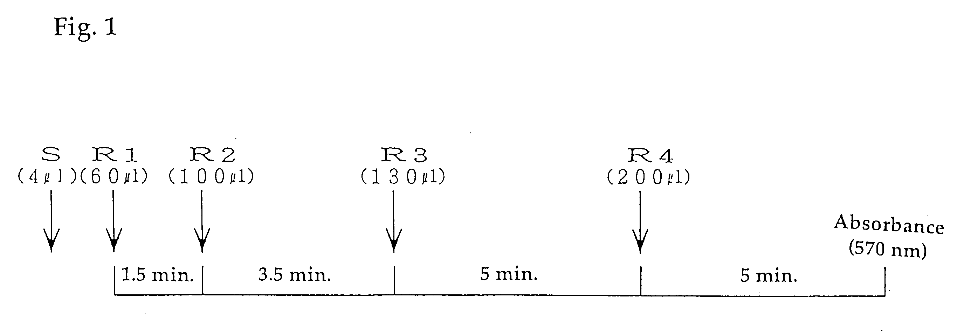

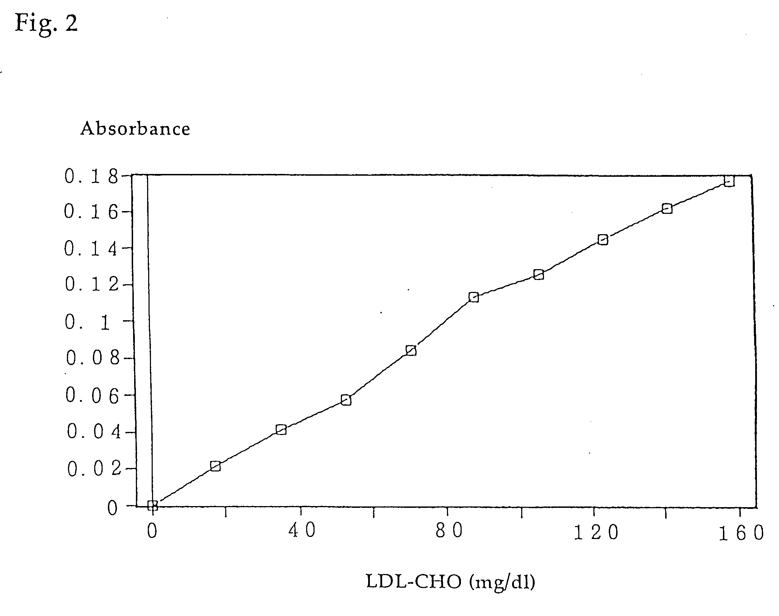

The following reagents were prepared:
- Reagent 1 (R1):: Antihuman apo-B goat serum
- Reagent 2 (R2):: 20 % PEG 4000, 13 units/ml CE, 13 units/ml CO, 1.0 unit/ml POD, 0.2 mg/ml N-(2-hydroxy-3-sulfopropyl)-3,5-dimethoxyaniline, 0.05 % TRITON X-100
- Reagent 3 (R3):: 1 % TRITON X-100, 0.11 mg/ml 4-aminoantipyrin (color former)
- Reagent 4 (R4):: 7M Guanidine hydrochloride, 1M guanidine thiocyanate
A series of samples were prepared by diluting human blood serum (S) containing 158.4 mg/dl of cholesterol in the LDL fraction, and an absorbance due to cholesterol in the LDL fraction in each sample was measured using Hitachi 7070 type automatic analyzer according to the analysis pattern of Fig. 1. The measured absorbance values are plotted against the concentrations of cholesterol in the LDL fractions (LDL-CHO). The results are shown in Fig. 2.

### Example 2

Cholesterol in the LDL fraction was measured by the method of the present invention and compared with the value obtained by the conventional quantitative analysis.

Using the same reagents as those used in Example 1, with 19 samples of fresh human blood serum (S), an amount of cholesterol was determined by the same automatic analyzer as used in Example 1 according to the same analysis pattern as in Example 1 (Fig. 1).

Separately, cholesterol in the LDL fraction of each of the same samples of the blood serum was quantitatively analyzed by the conventional precipitation method disclosed in Clin. Chem., 18, 499, 1972, the disclosure of which is hereby incorporated by reference, and the measured amounts were compared with the above amounts according to the present invention.

The results are shown in Table 1.

From the above results, it is understood that the measured amounts of the present invention well correlates with those obtained by the conventional Friedewald method (a correlation factor r of 0.987), and cholesterol in the LDL fraction can be quantitatively analyzed.

**Table 1**

| Blood serum No. | Present invention method (mg/dl) | Friedewald method (mg/dl) |
|---|---|---|
| 1 | 108.3 | 121.9 |
| 2 | 163.5 | 181.4 |
| 3 | 78.3 | 85.5 |
| 4 | 91.5 | 96.8 |
| 5 | 145.1 | 160.1 |
| 6 | 131.5 | 128.4 |
| 7 | 49.8 | 59.0 |
| 8 | 136.7 | 150.2 |
| 9 | 150.3 | 162.4 |
| 10 | 129.5 | 137.0 |
| 11 | 92.0 | 92.9 |
| 12 | 129.4 | 124.4 |
| 13 | 119.5 | 122.4 |
| 14 | 153.1 | 164.2 |
| 15 | 195.0 | 207.2 |
| 16 | 116.1 | 123.5 |
| 17 | 116.7 | 134.3 |
| 18 | 186.4 | 203.3 |
| 19 | 183.5 | 190.9 |

## Claims

1. A method for the direct quantitative analysis of a component contained in a specific lipoprotein fraction which is present in a biological sample, comprising steps of:
agglutinating said specific lipoprotein fraction,
leading a component, which is contained in lipoprotein fractions other than said specific lipoprotein fraction and is the same as said component that is contained in said specific lipoprotein fraction and to be analyzed, to a different reaction system which does not take part in said quantitative analysis;
dissolving said once agglutinated specific lipoprotein fraction,
subjecting said specific lipoprotein fraction to a quantitative reaction,
and
measuring a degree of a change caused by said quantitative reaction to determine an amount of said component in said specific lipoprotein fraction.

2. The method for the direct quantitative analysis according to claim 1, wherein said specific lipoprotein fraction is a low density lipoprotein fraction.

3. The method for the direct quantitative analysis according to claim 1, wherein said component to be quantitatively analyzed is cholesterol.

4. The method for the direct quantitative analysis according to claim 1, wherein the measurement of said degree of a change is carried out after saturation of a reaction or after a certain period of time from the start of a reaction, or optionally after the termination of a reaction.
